(19) Europäisches Patentamt

European Patent Office

Office européen des brevets

(11) Veröffentlichungsnummer: **0 247 571 B1**

(12) **EUROPÄISCHE PATENTSCHRIFT**

(45) Veröffentlichungstag der Patentschrift: **28.08.91**

(21) Anmeldenummer: **87107619.6**

(22) Anmeldetag: **25.05.87**

(51) Int. Cl.⁵: **A61M 25/02**

(54) **Fixiersystem zur Befestigung von Kathetern, Kanülen oder dgl. auf der Hautoberfläche.**

(30) Priorität: **28.05.86 DE 3617882**
**22.12.86 DE 3643985**
**16.04.87 DE 3713114**

(43) Veröffentlichungstag der Anmeldung:
**02.12.87 Patentblatt 87/49**

(45) Bekanntmachung des Hinweises auf die
Patenterteilung:
**28.08.91 Patentblatt 91/35**

(84) Benannte Vertragsstaaten:
**AT BE CH DE ES FR GB GR IT LI LU NL SE**

(56) Entgegenhaltungen:
**US-A- 3 612 265**
**US-A- 3 918 446**
**US-A- 4 122 857**
**US-A- 4 333 468**

(73) Patentinhaber: **LTS Lohmann Therapie-
Systeme GmbH & Co. KG
Irlicherstrasse 55
W-5450 Neuwied 12(DE)**

(72) Erfinder: **Becher, Frank
Hohenfelder Str. 5
W-5400 Koblenz(DE)**

(74) Vertreter: **Neidl-Stippler, Cornelia, Dr.
Rauchstrasse 2
W-8000 München 80(DE)**

## Beschreibung

Die Erfindung betrifft ein Fixiersystem zur Befestigung von Kathetern, Kanülen oder dergleichen mit einer mit einer Hautadhäsionsschicht beschichteten Folie mit Abdeckfolie und einem mit auf der Hautfolie klebenden Haftkleberschicht ausgerüsteten Oberpflaster mit Oberpflasterabdeckschicht.

Unter Kanülen bzw. Kathetern sollen hier alle "Sonden" verstanden werden, wie sie beispielsweise zur Ein-/Ableitung von Körperflüssigkeiten in/aus Gefäßen verwendet werden, wie Nadeln, Kanülen, Katheter, Meßsonden (bzw. für Messung bestimmter Körperparameter wie Sauerstoffpartialdruck oder dergleichen), Infusionseinrichtungen u.ä.. Die Sicherung von Kanülen am Körper muß beispielsweise während der Dialyse bei Patienten bei längeren Infusionen zur Vermeidung von mechanischer Belastung derselben erfolgen.

Einrichtungen zur Fixierung von Kanülen und Kathetern sind bekannt. So ist aus der EP-B1-76 896 und der EP-A1-121 679 ein "medizinischer Verband zur Fixierung einer Sonde", der einen an der Haut und am aus der Haut herausragenden Teil der Sonde gleichzeitig durch Pflaster befestigbaren Verbandabschnitt aufweist, bekannt geworden. Dieser Verbandabschnitt ist mit einem rohrförmig ausgebildeten Fortsatz ausgerüstet, der als Stütze oder Aufnahme für den aus der Haut herausragenden Teil der Sonde dient.

Die DE-U-82 04 827.4 beschreibt eine zwei Klemmplatten aufweisende Halteeinrichtung, die die Kanüle aufnimmt und sodann mit Pflaster auf der Haut festgeklebt wird.

Die DE-A-32 12 458 betrifft ein Bindensystem für an männlichen Personen äußerlich anzubringende Katheter und bezieht sich insbesondere auf die geometrische Ausgestaltung von Pflasterstreifen.

Die DE-C-29 47 427 betrifft eine Venenkatheterbandage, die mittels eines Pflasters mit einer wasserdichten und luftdurchlässigen haftklebend ausgerüsteten Folie, die ggf. eine feuchtigkeitsaufsaugende Schicht an der hautabgewandten Seite aufweisen kann, eine Kanüle mit Sperrventil auf der Haut festklebt - als bevorzugte Abmessungen werden 95 x 60 mm für das Pflaster genannt.

Die DE-A- 31 05 187 beschreibt ein Kanülenfixierpflaster mit einem ausgestanzten Pflastersegment, das nach Einstechen der Kanüle auf der Kanüle festgeklebt wird und derart ein Herausziehen der Kanüle verhindert, während das restliche Pflaster auf die Haut geklebt wird.

Die EP-A1-116 526 betrifft ein anderes Befestigungsprinzip für Kanülen an der Haut, bei dem eine Katheterhalteeinrichtung mittels einer durch die Haut des zu behandelnden Patienten gestochenen Nadel befestigt wird.

Bei besonderen Fällen wird der Katheter zur Sicherung seiner Lage gelegentlich sogar an der Haut angenäht.

Aus der US-A-3 918 446 (Buttaravoli) ist ein Fixiersystem zur Befestigung von Kathetern oder dergleichen mit einem Hauptpflaster mit Abdeckfolie und einem auf die Hautfolie klebbaren Oberpflaster mit Oberpflasterabdeckschicht bekannt geworden.

Das Unterpflaster besteht dabei aus einem Polyurethanschaum, ist undurchsichtig und nicht luftdurchlässig.

Zur Inspektion der Infusionsnadel werden Löcher im Oberpflaster vorgeschlagen.

Die US-A-4 122 857 (Haerr) bezieht sich auf eine Fixiereinrichtung für Schläuche und dergleichen auf der Haut eines Patienten, mit der ein Unterpflaster und ein Oberpflaster, die ggf. einseitig miteinander verbunden sein können und im wesentlichen gleich groß sind, vorgeschlagen werden. Der zu befestigende Schlauch wird dabei auf das auf der Haut aufgeklebte Unterpflaster aufgelegt und mittels eines Oberpflasters auf dem Unterpflaster aufgeklebt. Es wird vorgeschlagen, bakterizid ausgerüstete Materialien einzusetzen.

Aus der US-A-4 333 468 (Geist) ist ein Kanülenstützkörper bekannt geworden, der zur Aufnahme einer Kanüle oder dergleichen eingerichtet ist, wobei dieser eine Basisplatte und eine sich nach oben öffnende Aufnahmevorrichtung aufweist. Es handelt sich dort um eine mechanisch verriegelbare Anordnung, die inflexibel - vom Gelenk des Kanülenstützkörpers abgesehen - ist.

Unter der Bezeichnung POROFIX-Kanülenpflaster werden Pflasterstreifen zur Fixierung von Kanülen vertrieben; diese bestehen aus Heftpflaster mit einem Schlitz, die insbesondere bei der Fixierung von Strauß-Kanülen bei Infusionen helfen.

Die bekannten Kanülenfixierpflaster sind insofern verbesserungsfähig als bei den bisher bekannten Fixierpflastern Hautfolien bzw. Hautpflaster verwendet wurden, die nicht wasserdampfdurchlässig waren, so daß eine Mazeration der Hautoberfläche stattfinden konnte.

Ferner waren die Hautfolien nach dem Stand der Technik bisher nicht durchsichtig, so daß das örtlich bestimmte Setzen einer Infusionskanüle durch das Pflaster hindurch nicht möglich war, da die Einstichstelle nicht durch das Pflaster betrachtet werden konnte.

Bei den bekannten Kanülenfixiereinrichtungen war es auch nachteilig, daß die Einstichstelle und die darunter liegenden Hautpartien im Einstichkanal vor Infektionen durch Bakterien, die von der Hautoberfläche anwandern schlecht geschützt waren. Man versuchte zwar, dieses Problem durch Desinfektion der Haut vor dem Einstich zu umgehen; die Desinfektion umfaßt jedoch nur die oberen Hautschichten und kann nicht verhindern, daß Bakterien von außen in den Einstichkanal eindringen können.

Eine gute Fixierung der Kanüle, um ein Verrutschen der Kanüle über einen längeren Zeitraum zu verhindern, ist wesentlich.

Die Anordnungen nach dem Stand der Technik verwenden entweder sehr aufwendige Apparate zur Kanülenfixierung, die mit Heftpflaster auf die Haut geklebt werden oder es werden sehr einfache Pflaster mit Einschnitten oder dergleichen eingesetzt, die keine zufriedenstellende längere Fixierung der Kanülen ermöglichen.

Es ist demzufolge Aufgabe der Erfindung, ein verbessertes Kanülenfixiersystem zu schaffen, das auch bei länger liegenden Kanülen, wie bspw. "Shunts" bei DialysePatienten, die Einstichstelle vor Infektionen schützt und bei guter Fixierung gleichzeitig eine Beschädigung der Hautschichten vermeidet, wobei eine gute Inspektion der Einstichstelle möglich sein soll.

Die Aufgabe wird erfindungsgemäß durch ein Fixiersystem zum Befestigen von Kathetern, Kanülen oder dergleichen, mit einer mit einer Hautadhäsionsschicht beschichteten Hautfolie mit Abdeckfolie und einem mit auf der Hautfolie klebenden Haftkleberschicht ausgerüsteten Oberpflaster mit Oberpflasterabdeckschicht, gelöst, wobei die Hautfolie ein schmiegsames wasserdampfdurchlässiges und bakteriendichtes, durchsichtiges Inzisionsfolienmaterial aus synthetischem oder natürlichem Polymer ist, und daß gegebenenfalls auch das Oberpflaster und dessen Haftklebeschicht durchsichtig sind.

Inzisionsfolien sind, wie bspw. in : Wilson, Kohn "Verbandstoffe und Krankenpflegeartikel", Deutscher Apotheker Verlag Stuttgart, 1983, beschrieben, selbstklebende Operationsfolien, die bakteriendicht und durchsichtig sind.

Durch die Verwendung dieses Materials ist es möglich, bspw. Infusionsnadeln durch die Inzisionsfolie zu setzen, da durch diese eine Betrachtung der Einstichstelle möglich ist und die mit der Inzisionsfolie beklebte Haut sich aufgrund der dünnen Folie im wesentlichen verhält wie Haut ohne Inzisionsfolie, also in ähnlicher Weise zusammengeschoben bzw. angehoben werden kann.

Eine bevorzugte Ausgestaltung der Erfindung ist dadurch gekennzeichnet, daß die Schichten sandwichartig übereinander gelegt und einseitig in einem Teilbereich ohne Zwischenfolie oder durch eine Zwischentrennfolie mineinander verbunden sind, wobei die Oberpflasterabdeckfolie zu einer Zwischentrennfolie zwischen der Hautfolie und der Haftklebeschicht des Oberpflasters wird.

Weiterhin ist es vorteilhaft, wenn bei einem erfindungsgemäßen Fixiersystem die Inzisionsfolie bspw. ein natürliches oder synthetisches Polymer wie vernetztes Kollagen, Polyurethan od. dgl. ist.

Falls Oberpflaster als auch Hautfolie durchsichtig ausgebildet werden, ist es möglich, auch bei längerem Fixieren einer Kanüle/Katheters etc. die Lage desselben ständig durch Inspektion der Außenseite des Kanülenfixiersystems zu überprüfen, ohne daß eine Entfernung oder Öffnung des Fixiersystems, was mit Infektionsrisiken bzw. Verletzungsrisiken für den Patienten verbunden ist, notwendig ist. Durch diese vorteilhafte Ausgestaltung können in vielen Fällen Kanülenwechsel umgangen werden.

Wenn die Hautfolie flächenmäßig größer als das Oberpflaster ist, wird durch die größere Haftfläche eine sehr gute Anhaftung der Hautfolie an der Haut erzielt und es kann nun ein Oberpflaster mit sehr stark eingestelltem Klebstoff in der Haftkleberschicht, der sogar ggf. zu Verletzungen der Hautschicht führen würde, falls er auf der Haut eingesetzt würde, auf die Hautfolie geklebt werden. Dabei ist es sinnvoll, wenn das Oberpflaster wesentlich kleinflächiger ist als das Unterpflaster, um ggf. eine Matzeration der Haut durch Luft- und Feuchtigkeitsausschluß zu verhindern, falls ein Oberpflaster mit einer schlechten Luft- und Feuchtigkeitsleitung eingesetzt wird.

In dem Fall ist es vorteilhaft, wenn die Haftklebeschicht des Oberpflasters stärker an der Hautfolie als die Hautadhäsionsschicht und der Haut haftet.

Bevorzugt weist die Oberpflasterabdeckfolie eine Trennlinie und ggf. zwei Abziehhilfen, für jeden Zwischentrennfolienabschnitt eine, auf.

Die Hautfolie kann in einer bevorzugten Ausgestaltung der Erfindung ein- oder beidseitig wirkstoffbeschichtet, bevorzugt mit einem bakteriziden Wirkstoff oder ähnl., sein. Zur Verhinderung der Ablösung der Ränder der Hautfolie von der Haut kann die Hautfolie zusätzlich zur Hautadhäsionsschicht einen umlaufenden, stärker haftklebenden Klebemittelrand aufweisen.

Das Oberpflaster kann perforiert sein. Dadurch kann sichergestellt werden, daß bei evt. notwendiger Aufbringung eines Lösemittels auf das Oberpflaster eine leichte Entfernung des Oberpflasters durch Abstumpfen der Kleberschicht durch das Lösungsmittel sichergestellt wird.

Nach dieser Lösungsmittelbehandlung ließe sich das Oberpflaster abziehen und die Kanüle entfernt werden. Anschliessend kann die Hautfolie entfernt werden. Durch die Perforation kann ferner einer Mazeration darunterliegender Hautflächen vorgebeugt werden.

Zum erfindungsgemäßen Fixiersystem gehört bei einer besonders bevorzugten Ausführungsform ein bevorzugt einen Klebstoff bildendes elastisches Polymer bzw. dessen Ausgangsmaterial, das auf der Hautfolie und der Kanülenoberfläche haftet und nach Aufbringen auf Hautfolie und Einstichstelle eine bakteriendichte Versiegelung der Einstichstelle gemeinsam mit der Hautfolie bildet. Dies hat auch zur Folge, daß keine Körperflüssigkeit unter dem Pflaster hervortritt, also die Einstichstelle nach außen versiegelt ist, was bei Patienten, die unter Krankheiten leiden, die durch Kontakt mit Blut übertragen werden können, wie Hepatitis oder AIDS, sehr sinnvoll sein kann.

Das erfindungsgemäße Fixiersystem kann bei einer weiteren Ausführungsform der Erfindung ferner einen Kanülenstützkörper und ferner eine an die Außenform einer Kanüle angepaßte Aufnahmeöffnung derart, daß die Kanüle bei auf der Hautfolie aufgesetztem Kanülenstützkörper in der Aufnahmeöffnung in einer vorherbestimmten Winkelposition fixiert ist, aufweisen. Dabei kann der Kanülenstützkörper auch eine ggf. haftklebend ausgerüstete Auflagefläche besitzen, mit der er auf die Hautfolie aufgesetzt und bspw. während der Einsetzens der Kanüle fixiert werden kann: Diese Ausführungsform ist dann besonders vorteilhaft, wenn ein Festkleben des Oberpflasters an der Kanüle, was beim Entfernen der Kanüle unter Umständen Zu Schmerzen beim Patienten führen könnte, vermieden werden soll. Die Befestigung der Kanüle erfolgt in diesem Fall durch Reibschluß in der Aufnahmeöffnung des Kanülenstützkörpers.

Vorteilhafterweise ist der Kanülenstützkörper ein Formkörper aus elastischem Material, bevorzugt einem Polymer, das ggf. durchsichtig ist. Dafür eignen sich bspw. Silikongummi oder andere Polymere.

Der Kanülenstützkörper kann sowohl über eine Haftklebeschicht, mittels eines Klebstoffes oder durch Überkleben mit dem Oberpflaster in seiner Position fixiert werden.

Bei dem erfindungsgemäßen Fixiersystem mit zwei getrennten Pflastern aus Abdeckfolie, Hautadhäsionsschicht und Hautfolie sowie Oberpflasterabdeckfolie, Haftklebeschicht und Oberpflaster wird zunächst die Hautfolie, die eine Inzisionsfolie ist, von ihrer Oberflächenabdeckschicht gelöst und auf die mit einer Kanüle zu versehende Hautfläche aufgeklebt. Anschliessend wird die Inzisionsfolie mittels der Kanüle durchstochen. Das Herausstanzen eines Stückes Inzisionsfolie durch die Injektionsnadel und Einschleusen desselben in den Einstichkanal kann umgangen werden, indem eine geeignete halbstumpfe Kanülenform gewählt wird, durch die lediglich ein hufeisenförmiger "Lappen" ausgestanzt wird, der mit einer Seite an der Folie verbunden bleibt; eine andere vorteilhafte Alternative besteht darin, daß eine Kanüle mit zurückziehbarem Mitteldorn verwendet wird; eine einfache Möglichkeit der Reinigung besteht auch darin, daß vor Einbringung von Flüssigkeiten in den Körper die Innenseite der Kanüle beispielsweise durch Erzeugung eines Unterdrucks auf der vom Körper abgewandten Seite, Ansaugen von Resten von ausgestanzten Abschnitten und Gewebeteilen gereinigt wird.

Eine besonders bevorzugte Ausführungsform mit wirkstoffbeladener Inzisionsfolie ist insbesondere dann sinnvoll, wenn das erfindungsgemäße Kanülenfixierpflaster lange auf der Haut bleibt. Das gleiche gilt bei Patienten, bei denen sich eine Infektion besonders negativ auswirken kann, weil häufig eine Kanüle in ihr Gefäß eingeführt wird, z.B. bei Dialyse-Patienten; bei diesen wird ein sog. Shunt, eine Verbindung zwischen Arterie und Vene, hergestellt, wo bei jeder Dialyse eine neue Kanüle gesetzt wird. Auch eine an der Oberseite der Hautfolie zusätzlich oder alternativ dazu mit bakteriziden Wirkstoffen beschichtete Inzisionsfolie würde eine Wanderung von Bakterien an der körperabgewandten Einstichstelle zu dieser weitgehend unterbinden können.

Bei einer weiteren besonders bevorzugten Ausführungsform des erfindungsgemäßen Fixierpflasters sind der Oberpflasterabschnitt und der Hautfolienabschnitt, getrennt durch eine ggf. beidseitig haftfähige Zwischentrennfolie, übereinander angeordnet. Es ist bevorzugt, wenn die Zwischentrennfolie eine Trennlinie aufweist, die einen größeren und einen kleineren Flächenabschnitt definiert. Dabei ist es sinnvoll, wenn die abziehbaren Zwischentrennfolienteile und Abdeckfolien Abziehhilfen aufweisen.

Zunächst wird die die Hautfolie hautseitig abdeckende Abdeckfolie entfernt (eventuell unter Verwendung einer Abziehhilfe) und das verbleibende sandwichartige Pflastersystem als Ganzes auf die Haut aufgeklebt. Anschließend wird ein Teil des oder das ganze Oberpflaster(s) gemeinsam mit der Zwischentrennfolie von der Hautfolie abgezogen. Ein Abschnitt der Zwischentrennfolie wird entlang einer vorbereiteten Trennlinie vom Trennzwischenfolienrest abgetrennt und vom Oberpflaster gelöst. Die nun freiliegende Haftklebeschicht wird auf die Hautfolie aufgeklebt. Anschliessend wird der Rest der Zwischentrennfolie gemeinsam mit dem durch ihn abgedeckten nicht angeklebten Oberpflasteranteil von der Hautfolie weggeklappt, so daß die Hautfolie frei über der Einstichstelle liegt. Es erfolgt der Einstich. Die Zwischentrennfolie wird nun vollständig entfernt - ggf. mit einer zweiten Abziehhilfe - und das Oberpflaster über die Kanüle geschlagen, so daß es in etwa hufeisenförmig auf dem unteren Pflaster aufliegt und an der offenen Seite des Hufeisens die Kanüle herausragt. (Selbstverständlich kann die Reihenfolge der Schritte auch geändert werden, bspw. zuerst die Kanüle gesetzt und anschließend das Oberpflaster aufgeklebt werden).

Zur Vermeidung von Mazerationserscheinungen und um das Ablösen des Oberpflasters durch Lösungsmittel zu erleichtern, kann es sinnvoll sein, wenn das Oberpflaster perforiert ist.

Mit Hilfe des erfindungsgemäßen Kanülenfixierpflasters wird die Kanüle in einer durch Hautfolie und Oberpflaster gebildeten Tasche eingeklebt und somit ein Kontakt der Kanüle mit der Haut weitgehend vermieden.

Zur Vermeidung des Ausstanzens eines Hautfolienanteils beim Einstich der Kanüle kann die Kanüle auch außerhalb des Fixiersystems, an einer Kante desselben, gesetzt werden, so daß die verbesserte Fixierung der Kanüle durch das erfindungsgemäße Fixiersystem ermöglicht wird, wobei eine vollständige Abdeckung des Einstichbereiches durch die Hautfolie nicht erfolgt, sondern alleine die Fixierwirkung des Fixierpflastersystems verwendet wird.

Die einzelnen Bestandteile des Fixiersystems, wie das Unterpflaster, das Oberpflaster sowie der auf diese abgestimmte Klebstoff und der Kanülenstützkörper sind erfindungswesentlich.

Im folgenden wird die Erfindung näher anhand der beiliegenden Zeichnung erläutert. Dabei zeigt:

Fig. 1     ein erfindungsgemäßes System mit getrenntem Oberpflaster und Hautfolie;

Fig. 2     einen Längsschnitt durch eine sandwichartige Oberpflaster-Hautfolien-Kombination gemäß der Erfindung;

Fig. 3     das Pflaster der Fig. 2 in der Draufsicht im Einsatzzustand;

Fig. 4     das Pflaster der Figuren 2 und 3 vor Überschlagen des Oberpflasters, mit aufgebrachtem Klebstoff; und

Fig. 5     eine Draufsicht auf eine erfindungsgemäße Oberpflaster-Abdeckschicht-Kombination mit Abziehhilfe und Trennlinie.

Fig. 6     eine Ansicht eines erfindungsgemäßen Fixiersystems mit Kanülenstützkörper; und

Fig. 7     das erfindungsgemäße Fixiersystem der Fig. 6 entlang der Linie VII-VII' geschnitten.

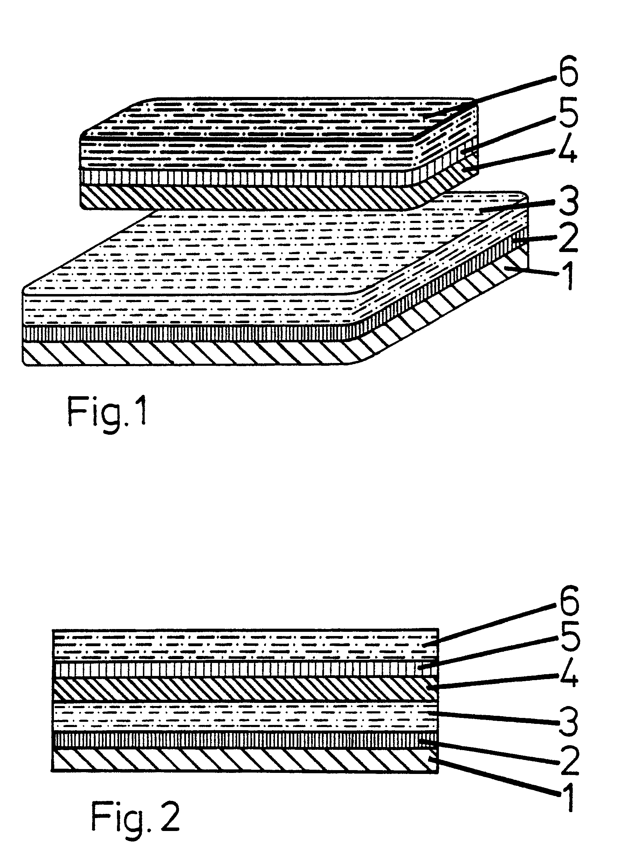

Wie in Fig. 1 gezeigt, besteht eine bevorzugte Ausführungsform des erfindungsgemäßen "Unterpflasters" aus einer Abdeckfolie 1, einer Hautadhäsionsschicht 2 und einer Hautfolie 3, die bevorzugt Luft- und Wasserdampf-durchlässig, ggf. auch bakterizid ausgerüstet sein kann. Das "Oberteil" des Fixiersystems besteht aus einem Oberpflaster 6, einer Haftklebeschicht 5 und einer Oberpflasterabdeckfolie 4, ggf. mit Abziehhilfe. Das in Fig. 1 dargestellte Fixiersystem kann die Kanüle 8 fixieren, die nach Aufkleben der Hautfolie auf die Haut gesetzt, ggf. mit einem bakteriendichten Klebstoff 7 versiegelt und anschliessend vom auf der Hautfolie 3 aufgeklebten Oberpflaster 6 befestigt wird.

In Fig. 2 ist eine sandwichartige Variante des erfindungsgemäßen Fixiersystems dargestellt, bei welcher die gleichen Schichten, wie für Fig. 1 beschrieben, übereinanderhaftend vorliegen, allerdings ist die Oberpflasterabdeckschicht 4 hier zu einer Zwischentrennfolie geworden.

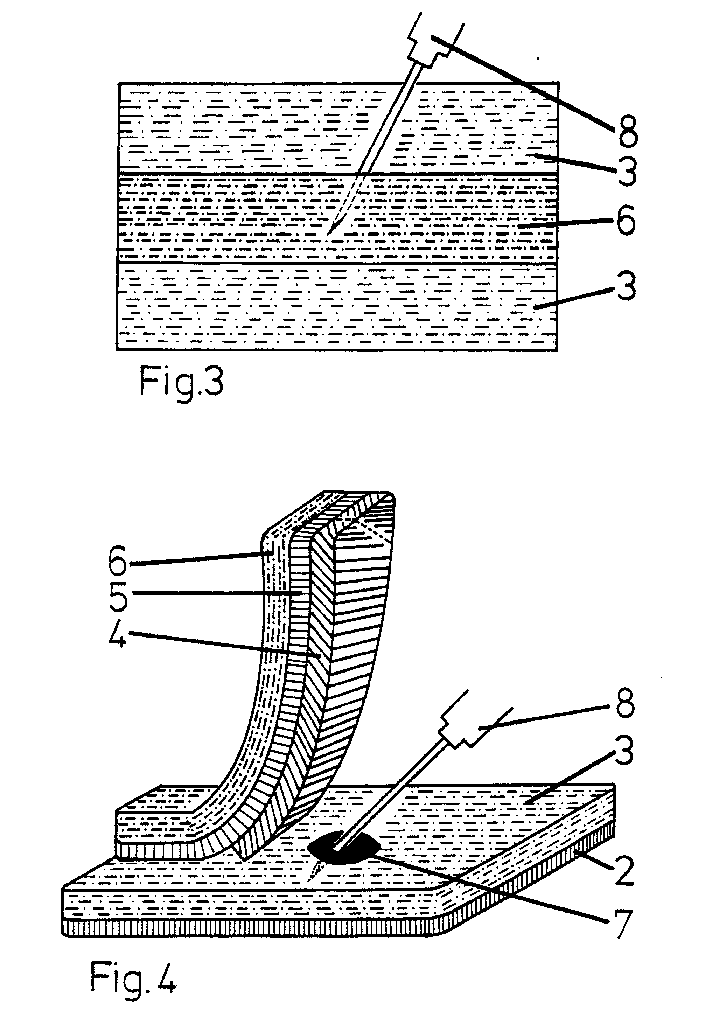

Im Fig. 3 ist das in Fig. 2 dargestellte Pflaster im auf die Haut aufgebrachten Zustand gezeigt: auf einer großflächigen Hautfolie 2 ist ein kleineres Oberpflaster 6 aufgeklebt, das die Kanüle 8 in ihrer Lage fixiert.

In Fig. 4 ist die Kanüle 8 bereits durch die aufgeklebte Hautfolie gestochen und das Oberpflaster mit einem von Zwischentrennfolie befreiten Abschnitt auf die Hautfolie 3 aufgeklebt. Der bakteriendichte Klebstoff 7 ist um die Einstichstelle auf der Hautfolie 3 und dem freien Kanülenende unter Bildung einer folienartigen Versiegelung aufgebracht. Der noch mit Zwischentrennfolie 4 versehene Oberpflasterabschnitt 3 ist zurückgeklappt und weist an seinem Ende eine Abziehhilfe auf, welche das Abnehmen des restlichen Zwischenfolienstückes erleichtert. Als nächster Arbeitsgang ist nun mittels der Abziehhilfe die Zwischentrennfolie 4 von dem Rest des Oberpflasters 6 zu entfernen, das Oberpflaster über die Kanüle 8 zu schlagen und festzukleben.

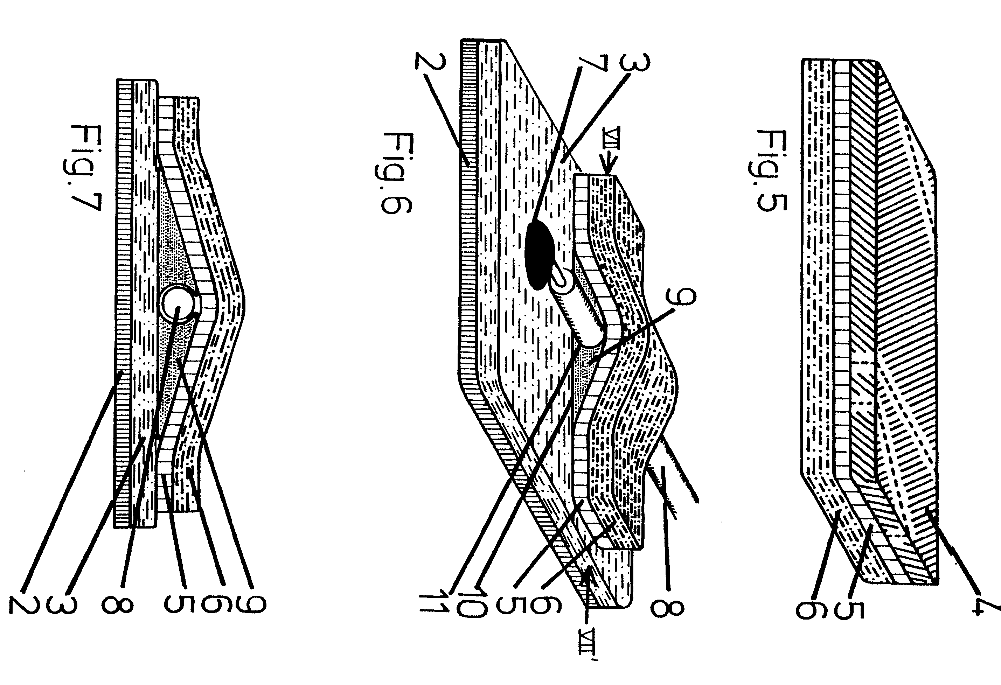

In Fig. 5 ist der in Fig. 4 aufgeschlagen gezeigte Oberflächenabschnitt, von der Zwischentrennfolienseite 3 her gesehen, gezeigt. Deutlich erkennt man zwei Abziehhilfen sowie eine Trennlinie, entlang derer zunächst ein Teilabschnitt der Zwischentrennfolie abgezogen und das Oberpflaster 6 sodann auf die Hautfolie 3 aufgeklebt werden kann.

In Fig. 6 ist eine Fortbildung des erfindungsgemäßen Fixiersystems gezeigt, bei dem ein aus einem elastischen Polymer hergestellter Kanülenstützkörper 9 mit einer Auflagefläche 10, die hier haftklebend ausgerüstet ist, auf der Hautfolie 3 aufgeklebt ist. Im Kanülenstützkörper 9 ist eine Aufnahmeöffnung 11 vorgesehen, in die die Kanüle 8 reibschlüssig eingesetzt werden kann. Bei der hier dargestellten Ausführungsform kann die Kanüle 8 durch einen Schlitz in einer elastischen Begrenzungswand der Aufnahmeöffnung 11 eingedrückt werden und wird durch die elastisch in ihre Ruhelage zurückkehrenden Kanülenstützkörperwände gehalten.

In Fig. 7 ist das Fixiersystem der Fig. 6 entlang der Linie VII-VII' geschnitten dargestellt, Hier ist ein Oberpflaster 6 zur besseren Befestigung der Kanüle 8 über den Kanülenstützkörper 9 geklebt; bei dieser Ausführungsform kann auf eine haftklebende Beschichtung der Hautauflagefläche 10 verzichtet werden.

## Patentansprüche

1. Fixiersystem zum Befestigen von Kathetern, Kanülen oder dergleichen, mit einer mit einer Hautadhäsionsschicht (2) beschichteten Hautfolie (3) mit Abdeckfolie (1) und einem mit auf der Hautfolie klebenden Haftkleberschicht (5) ausgerüsteten Oberpflaster (6) mit Oberpflasterabeckschicht (4), dadurch gekennzeichnet, daß die Hautfolie (3) ein schmiegsames wasserdampfdurchlässiges und bakteriendichtes, durchsichtiges Inzisionsfolienmaterial aus synthetischem oder natürlichem Polymer ist, und daß gegebenenfalls auch das Oberpflaster (6) und dessen Haftklebeschicht (5) durchsichtig sind.

2. Fixiersystem nach Anspruch 1, dadurch gekennzeichnet, daß das Inzisionsfolienmaterial Polyurethan oder vernetztes Collagen ist.

3. Fixiersystem nach einem der vorangehenden Ansprüche, dadurch gekennzeichnet, daß die Hautfolie (3) flächenmäßig größer als das Oberpflaster (6) ist.

4. Fixiersystem nach einem der vorangehenden Ansprüche, dadurch gekennzeichnet, daß die Haftklebeschicht (5) des Oberpflasters (6) stärker an der Hautfolie (3) haftet als die Hautadhäsionsschicht (2) an der Haut.

5. Fixiersystem gemäß einem der Ansprüche 1 bis 4, dadurch gekennzeichnet, daß die Oberpflasterabdeckfolie (4) eine Trennlinie und ggf. zwei Abziehhilfen, für jeden Abschnitt eine, aufweist.

6. Fixiersystem gemäß den Ansprüchen 1 bis 4, dadurch gekennzeichnet, daß die Hautfolie (3) zusätzlich einen umlaufenden, stark haftklebenden Klebemittelrand aufweist.

7. Fixiersystem gemäß einem der vorangehenden Ansprüche, ferner gekennzeichnet durch ein bevorzugt einen Klebstoff bildendes elastisches Polymer (7) bzw. dessen Ausgangsmaterial, das im Bereich der Einstichstelle auf der Hautfolie (3) und der Kanülenoberfläche haftet und derart eine bakteriendichte Versiegelung der Einstichstelle bildet.

8. Fixiersystem gemäß einem der vorangehenden Ansprüche, ferner gekennzeichnet durch einen Kanülenstützkörper (9) ggf. mit einer haftklebend ausgerüsteten Auflagefläche (10) und mit einer an die Außenform der Kanüle (8) angepaßten Aufnahmeöffnung (11) derart, daß die Kanüle (8) bei auf der Hautfolie (3) oder der Haut aufgesetztem Kanülenstützkörper (9) in der Aufnahmeöffnung (11) in einer vorherbestimmten Winkelposition fixiert ist.

9. Fixiersystem gemäß Anspruch 9, dadurch gekennzeichnet, daß der Kanülenstützkörper (8) ein Formkörper aus elastischem Material, bevorzugt einem Polymeren ist, das ggf.durchsichtig ist.

**Claims**

1. Fixing system for fastening catheters, cannulas etc., having a skin film (3) coated with a skin adhesion layer (2) and having a cover film (1) and an overplaster (6) finished with a pressure sensitive adhesive layer (5) adhering to the skin film and having an overplaster cover layer (4), characterized therein that the skin film (3) is a flexible, watervapour-permeable and bacteria-tight transparent incision film material consisting of synthetic or natural polymer and that optionally the overplaster (6) and its pressure sensitive adhesive layer (5) are transparent.

2. Fixing system according to claim 1, characterized in that the incision film material (3) is a crosslinked collagen or polyurethane.

3. Fixing system according to one of the preceding claims, characterized in that the surface of skin film (3) is larger than that of overplaster (6).

4. Fixing system according to one of the preceding claims, characterized in that the pressure sensitive adhesive layer (5) of overplaster (6) adheres more strongly to the skin film (3) than the skin adhesion layer (2) to the skin.

5. Fixing system according to one of the claims 1 to 4, characterized in that the overplaster cover film (4) has a separating line and optionally two removal aids, one for each portion.

6. Fixing system according to claims 1 to 4, characterized in that the skin film (3) additionally has an all-round, strongly pressure sensitive adhering adhesive border.

7. Fixing system according to one of the preceding claims further characterized by an elastic polymer (7) or its starting material preferably forming an adhesive, which adheres to the skin film (3) and the cannula surface in the region of the injection point and in this way forms a bacteria-tight seal for the insertion point.

8. Fixing system according to one of the preceding claims further characterized by a cannula support body (9), optionally with a contact adhesively finished support surface (10) and with a reception opening (11) adapted to the external shape of a cannula (8), in such a way that cannula (8) in the case of the cannula support body (9) being applied to the skin film (3) or skin is fixed in a pre-determined angular position in reception opening (11).

9. Fixing system according to claim 8, characterized in that the cannula support body (8) is a moulded article made from an elastic material, preferably a polymer, which is optionally transparent.

**Revendications**

1. Dispositif de fixation de cathéters, canules ou analogues comprenant une feuille (3) en contact avec la peau revêtue d'une couche adhérant à la peau (2) et munie d'une feuille de recouvrement (1) et un pansement supérieur (6) doté d'une couche autocollante (5) adhérant à la feuille en contact avec la peau et munie d'une couche de recouvrement (4), caractérisé en ce que la feuille en contact avec la peau (3) est une matière en feuille pour incision transparente, flexible, perméable à la vapeur d'eau et étanche aux bactéries constituée d'un polymère synthétique ou naturel, et en ce que, éventuellement, le pansement supérieur (6) et sa couche autocollante sont également transparents.

2. Dispositif de fixation selon la revendication 1, caractérisé en ce que la matière en feuille pour incision est du polyuréthane ou du collagène réticulé.

3. Dispositif de fixation selon l'une des revendications précédentes, caractérisé en ce que la feuille en contact avec la peau (3) a une surface plus grande que celle du pansement supérieur (6).

4. Dispositif de fixation selon l'une des revendications précédentes, caractérisé en ce que la couche autocollante (5) du pansement supérieur (6) adhère plus fortement à la feuille en contact avec la peau (3) que la couche d'adhérence (2) à la peau.

5. Dispositif selon l'une des revendications 1 à 4, caractérisé en ce que la feuille de recouvrement (4) du pansement supérieur présente une ligne de séparation et éventuellement deux dispositifs de dégagement pour chaque section.

6. Dispositif de fixation selon les revendications 1 à 4, caractérisé en ce que la feuille en contact avec la peau (3) présente en outre un bord périphérique adhésif fortement autocollant.

7. Dispositif de fixation selon l'une des revendications précédentes, caractérisé en outre par un polymère élastique (7) formant de préférence une colle ou sa matière de départ qui adhère à la feuille en contact avec la peau (3) et à la surface de la canule dans la zone du point de perforation et forme de ce fait une barrière étanche aux bactéries dans cette zone.

8. Dispositif de fixation selon l'une des revendications précédente, caractérisé en outre par un corps de support de canule (9), éventuellement avec une surface d'appui (10) dotée d'une couche autocollante et une ouverture réceptrice (11) adaptée à la forme externe de la canule (8) en sorte que la canule (8), grâce au corps de support installé sur la feuille en contact avec la peau ou sur la peau elle-même, soit fixée dans l'ouverture réceptrice (11) selon une position angulaire prédéterminée.

9. Système de fixation selon la revendication 8, caractérisé en ce que le corps de support de canule (8) est un corps moulé en matériau élastique,de préférence un polymère qui est éventuellement transparent.

Fig.1

Fig. 2

Fig.3

Fig.4

Fig.5

Fig.6

Fig.7